# EUROPEAN PATENT APPLICATION

(11) **EP 3 348 288 A2**
(43) Date of publication of application: **18.07.2018**
(21) Application number: 18151487.8
(22) Date of filing: 12.01.2018
(51) Int. Cl.: A61L 31/06, A61B 17/68

(54) **IMPROVED BONE FIXATION DEVICES**

(30) Priority: 13.01.2017 US 201762446028 P; 20.12.2017 US 201715849518
(71) Applicant: M.M.A. Tech, Ltd., 2201202 Nahariya (IL)
(72) Inventor: ELIAZ, Ari, 2201202 Nahariya (IL)
(74) Representative: J A Kemp

(57) **Abstract**

Intramedullary nails and other bone stabilization devices used to provide for bone fixation are made from a biocompatible polymer such as the pyromellitic, dianhydride (PMDA)-free, non-halogenated, thermosetting aromatic polyimide disclosed in U.S. Patent 6,686,437. The intramedullary nails of this invention can be secured to the bone by screws anywhere along their length. The intramedullary nails of the invention eliminate the need for imaging technology, permitting the surgeon more freedom to personalize the operation and support to the needs to the needs of the particular patient and avoid the need to expose the patient and medical personnel to hazardous x-rays and the like. The use of a polyimide polymer instead of a metal device of far higher modulus reduces bone fracturing and splintering and eliminates the risk of metallosis and metal poisoning. Ordinary commercial metal screws can also be exchanged with polyimide self - tapping screws providing a totally non-metal system.

## Description

The present invention relates to a bone stabilization device, in particular to intramedullary nails, plates and screws used to provide internal fixation of broken bones. Internal fixation is an operation in orthopedics that involves surgical insertion of implants (nails, plates and the like) to guide the healing process of a bone.

There are a variety of ways to treat bone fractures, in order to stabilize the bone while it heals. The most common alternative is a cast or splint applied to the outside of the limb in question, but such devices are subject to patient compliance, often a nuisance, and most importantly perhaps do not provide weight bearing while preventing the bone and limb from rotating. Consequently, fractures, particularly of long bones that bear weight, as well as complicated fractures, are commonly addressed by provision of an intramedullary nail or plate.

Intramedullary nails are long rods made of a high strength material that are typically inserted by the surgeon treating the fracture through the hollow marrow tunnel center of the bone, or medullary cavity of the fractured bone. In a sense, the nail becomes a sort of internal splint to stabilize the fracture. The nail has a mechanical advantage by being at the load-bearing axis. The intramedullary nail must be secured to the broken bone to stabilize it. This is often done by inserting screws through the bone and into pre-drilled holes in the nail. Rigid fixation prevents flexural motion across lines of fracture to enable healing and prevent infection.

Fixation plates are used in fully invasive or in minimally invasive percutaneous fractures and provide an alternative to manage these lesions. Minimally invasive percutaneous plating is inserted percutaneously through a small incision, thereby providing some stability and minimizing any intraoperative iatrogenic soft tissue damage. The plate is secured to the outer circumference of the bone (not inserted in the bone). This technique provides less stability than nails but can be used to treat complicated fractures. Prior art intramedullary nails have typically been used to provide fixation of long bones, typically leg and arm bones.

In a conventional intramedullary device, a long blank (rod or plate) is made of a material of high tensile strength and stiffness. Typical materials used for nails and plates have been Titanium, Stainless Steel (SS), PEEK/C. In these prior art systems, a rod, or blank, longer than it is wide, would be surgically inserted in the intramedullary cavity of the broken bone, such as a leg or arm bone, to provide internal fixation, as opposed to a cast which immobilizes the limb, but presents patient compliance and comfort issues.

A problem with the earlier designs was the failure to prevent collapse or rotation in inherently unstable fractures. This was addressed in prior art systems with the introduction of the concept of 'locking' of the nails using screws at least on each end of the nail (thus fixing the nail to the bony cortex and preventing rotation among the broken fragments), leading to emergence of locked intramedullary nailing, which is the standard today. A supplemental practice in the art of bone stabilization as practiced today is the use of Kirschner wire, or K-wire to at least provide temporary fixation of the bone. The use of such "K-wires" was pioneered by Martin Kirschner in the early 1900s, but they are now widely used in complex situations, such as the fixation and stabilization of fractures of the kneecap or olecranon. K-wires or K-pins are sterilized, sharpened smooth stainless steel inserts that can be used to stabilize complex fractures where several pieces of bone need to be stabilized one to the next.

Representative examples of prior art systems available today for bone fixation are set forth in U.S. Patent Nos. 9,101,417 and 9,562,549. In U.S. Patent No. 9,101,417 a composite bone implant is discussed, in which the nail or blank is provided with X-ray opaque material, because the nail must be properly positioned to permit the fixation with screws that must go through the predrilled holes in the nail. Thus, to ensure proper placement and alignment of the intramedullary nail, a common surgical practice is taking repeated or continuous X-rays of the fractured limb with the nail in place, and then adjusted, so as to provide for proper alignment of the device. In the '417 Patent, the plate or nail is made of a fiber reinforced polymer matrix, where the polymer is typically polyether ether ketone (PEEK). This polymer is reinforced with carbon fibers and the like. The nail or plate are provided with elements of an X-ray and radioscopically opaque material, typically a metal thread, so as to be able to visualize the alignment of the predrilled holes of the nail or blank.

U.S. Patent No. 9,562,549 is addressed to the screw used to secure the nail or blank to the bone, to prevent rotation and/or non-aligned healing. It too is described as made of carbon-reinforced polymer. The problem of proper location of the screw, vis-à-vis the intramedullary nail and the bone, is addressed in detail, with the screw being provided with a specific cutting edge that may improve interaction between the bone and the screw. Fundamentally, these current technologies, and virtually all prior art intramedullary devices, rely on the process of securing the blank or nail to the bone by the insertion of screws through predrilled and pre-threaded holes provided in the insert. The problems caused by proper location of the nail or blank within the bone, so that the predrilled holes line up with the portions of the bone selected to secure the device against rotation is substantial, and imposes a requirement, often of multiple or continuous X-rays or scans of the nail during it's insertion. The repeated and sustained and especially continuous scans pose a danger to not only the patient, but the medical team as well. Studies reflect a high rate of cancer in orthopedic doctors and medical team, which is are linked to the sustained and repeated exposure to X-rays and similar high energy imaging techniques.

Risks and complications of intramedullary devices can include bacterial colonization of the bone, infection, stiffness and loss of range of motion, non-union, mal-union, damage to the muscles, nerve damage and palsy, arthritis, tendonitis, chronic pain associated with thermal changes in environment effected by high thermal conductivity of the plates, screws, and pins, compartment syndrome, deformity, audible popping and snapping, and possible future surgeries to remove the hardware. Another risk is the Cut-out (antero-posterior deviation) phenomena of the metal screw in the nail's hole breaking the bone before final healing as a result of difference in material hardness of metal compared to bone. Another problem arising due to the use of composite materials is when drilling or screwing the nail or plate, provided from fiber reinforced composites in other than the precise correct direction (exactly into the predefined hole)- debris from carbon fibers may enter the body tissue, which is extremely dangerous. In this case, the area around the holes must be free of carbon fibers, which will weaken this location and create a non-uniform nail or plate with non-continuous fibers and with different strength along the device.

While as noted, certain devices are provided from fiber reinforced composites, typically these are highly expensive materials whose preparation, including detailed molding requirements, make wide use prohibitively expensive, and alternate devices made of less expensive materials are used. Clearly, such devices must be made of materials with high tensile strength, but sparingly light. As an alternative to highly expensive fiber reinforced composites, choices like stainless steel, and more typically titanium, developed in advance of the composite materials, are commonly selected as the basis for the intramedullary nail. This has significant consequences - since the nail must be secured to the bone in which it is inserted by means of screws drilled into the bone - the nail must be predrilled with holes, and those holes then located after insertion by X-ray or similar imaging technology. This is also true for the more expensive composite materials and all metal intramedullary nails. The screws must contact the intramedullary nail precisely, as they must line up with the predrilled holes provided. Drilling into the metal or composite rod at a spot other than the predrilled hole is not a suitable option - the drilling into metal generates temperatures much too high to be compatible with the organic material of the bone and muscles surrounding. Yet, often, the placement of the plate or nail is imperfect, or the preset holes in the rod require a placement that is not ideal. Additionally, fixation of the intramedullary nail requires the provision of a metal strap or frame exterior to the plate or nail.

Typically, stabilization of the bone fracture requires an incision through the skin and tissue at one end of the bone. The nail is inserted into the hollow marrow tunnel of the bone, but the screws must then be set and drilled into the cortex of the bone and then into the preset holes, fixing the framework. Thus, when the fracture is well-healed, the limb must be opened and the framework removed. This architecture is imposed by the need to prevent the intramedullary nail from moving, which is effected by the screws inserted through it. It would be advantageous if the surgical removal of the framework could be avoided, and the need to set the screws securing the intramedullary rod to the bone exactly opposite predrilled holes dispensed with. It would also be desirable if the need to image the nail by X-ray following or during its insertion, so as to precisely locate it to provide for alignment of the predrilled holes, could be avoided or reduced. Such imaging requirements complicate and prolong the surgery, and present complications that are well avoided.

Taking advantage of the unique properties of biologically compatible pure polyimide polymer MP-1™, commercially available from MMATECH Ltd. of Israel, a new and improved intramedullary nail is provided, which avoids many of the drawbacks of the prior art. The polymer, its preparation and characteristics are fully disclosed in U.S. Patent No. 6,686,437, and reference is made to that publication for further details. The '437 Patent discloses a pyromellitic, dianhydride (PMDA)-free, non-halogenated, thermosetting aromatic polyimide that may have the chemical formula 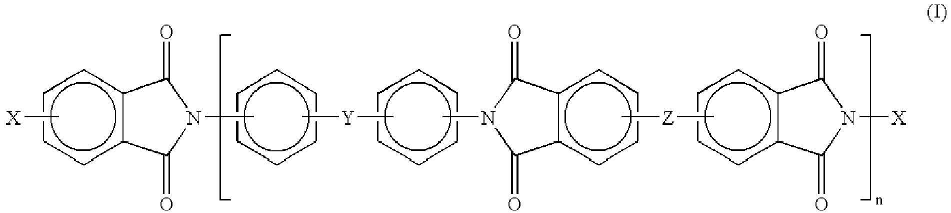 wherein:
Y and Z are each independently selected from the group consisting of a chemical bond between two adjacent aromatic rings, O, CO and substituted phenyl, where Y and Z can be linked to any free position on the adjacent aromatic ring;
X is selected from the group consisting of substituted allyl, vinyl, acetynyl, phenylethynyl and benzocyclobutenyl; and n represents an integer equal to or greater than 1, preferably n ranges from about 1 to about 100.
Preferably, the intramedullary device is prepared from a polymer of the above-described formula where X is selected from the group consisting of benzocyclobutenyl, acetynyl and phenylethynyl; Y and Z are each independently selected from the group consisting of a chemical bond between two adjacent aromatic rings and O; n represents an integer ranging from about 10 to about 100; and X, Y, and Z being linked to the adjacent aromatic rings in the 3, 3', 4 and 4' positions.

The intramedullary nail has the requisite strength, as reflected in the disclosure of U.S. Patent No. 6,686,437, but can be drilled into by conventional bone self-tapping screws used to secure the nail while in the body. Present nail requirements for precise placement are no longer required. In a preferred embodiment, the intramedullary nail is provided with screw indentations or abrasions along it length, since the screw can be directly drilled into the MP-1™ at various locations and different angles all the way along the intramedullary nail which transforms it into a robust solution. Since there is no need to provide pre-drilled holes in the nail or plate, the surgeon can drill anywhere along it, and does not have to aim into or at a specific spot. Accordingly, the surgeon is not dependent on X-ray guided adjustment, and the dangerous process of imaging the bone and nail during and after insertion can be dispensed with. Advantageously, in a process of bone fixation using the invention of this application, the entire process is X-ray free.

At the same time the need for preset screw holes and precise configuration is avoided, the need for complicated imaging technology is also reduced. The surgeon is now free to provide fixation at a point that is more precise and convenient to the surgeon, patient and offers superior fixation of the broken bone. Similarly, the problems presented by prior art metal intramedullary nails, including metallosis, bone breakage due to the large difference between the elastic modulus of metal and the much lower elastic modulus of bone (resulting in twisting and breaking of the bone) before complete healing can be achieved are eliminated. A further benefit is avoiding the need to remove the metal superstructure required for fixation of the intramedullary bone. This operation, which is conducted under total anesthesia, presents a risk to the patient. Using the biocompatible polyimide polymer of MP-1™ or a similar polymer permits use of a biocompatible polymer nail which need not be removed, and can safely stay in the body for a lifetime, improving the outcomes.

There is disclosed a bone stabilization device for insertion in the body of a patient requiring bone stabilization, said bone stabilization device comprising: a shaft comprised of a formable, pyromellitic, dianhydride (PMDA)-free, non-halogenated, aromatic polyimide thermosetting polymer which preferably exhibits approximately the tensile strength and stiffness of human bone, wherein said shaft is of a shape suitable for stabilization of said bone requiring stabilization, and is free of predrilled holes for bone screws.

The shaft material optionally has a tensile strength in the range 125 MPa - 175 MPa, preferably approximately 150 MPa.

The shaft material optionally has a tensile modulus (stiffness) in the range 3800 MPa - 4300 MPa, preferably approximately 4070 MPa.

The shaft material optionally has a strain to failure value in the range 1.5% - 2.5%, preferably approximately 2%.

The shaft material optionally has a fracture toughness in the range 3.5 MPa(m)^{1/2} - 4.5 MPa(m)^{1/2} , preferably 4 MPa(m)^{1/2}.

Optionally, said shaft is of a shape and size to be inserted in the intramedullary marrow tunnel of a long human bone, and is susceptible of being secured to the bone it stabilizes by bone self-tapping screws.

For example, the shaft may take a length in the range 10 cm to 50 cm, preferably 15 cm to 40 cm, more preferably 20 to 30 cm.

Optionally, said device is of a shape and size suitable to stabilize a partial fracture and is in the form of a plate or flange attached to the bone but not penetrating any cavity of the bone.

Optionally, said device comprises abrasion roughening at points along its length to aid in the driving of screws to secure said device to said bone to be stabilized.

There is disclosed a method of stabilizing a fractured bone comprising: affixing the abovementioned bone stabilization device to said bone to be stabilized by screwing said device to said bone with bone self-tapping screws at any point along the length and at any angle with respect to said device suitable for a surgeon affixing said device.

Optionally, said method does not require any imaging to determine proper placement of said device.

Optionally, said method is free of the provision of scaffolding or support elements necessary to hold said device in place relative to said bone.

Optionally, said method comprises inserting the device in the form of an intramedullary nail into the intramedullary marrow tunnel of the bone to be stabilized, and wherein said nail is secured by bone self-tapping screws screwed through said nail and into said bone cortex to be stabilized.

Optionally, said bone is partially broken, and said device is affixed by said bone screws to portions of said bone not within the zone of breakage of said bone.

Optionally, said bone is not a long bone, and said device is in the form of a plate or flange affixed to bone along said bone, so as to stabilize said bone while it heals.

Optionally, said self-tapping screws are prepared from a biocompatible polymer which need not be removed from the body.

Optionally, said method does not employ any materials of metal to be left in the body of a patient with said fractured bone.

There is disclosed a kit comprising: the bone stabilization device of any one of the preceding claims; and bone self-tapping screws that may be screwed at any point along the length and at any angle with respect to said device.

Optionally, said kit is free of scaffolding or support elements necessary to hold said device in place relative to said bone.

Optionally, said device is in the form of an intramedullary nail for insertion into the intramedullary marrow tunnel of the bone to be stabilized, and wherein said nail is securable by said bone self-tapping screws screwed through said nail and into said bone cortex to be stabilized.

Optionally, said device is in the form of a plate or flange affixable to bone along said bone, so as to stabilize said bone while it heals.

Optionally, said bone self-tapping screws are prepared from a biocompatible polymer which need not be removed from the body.

Optionally, said kit does not comprise any materials of metal to be left in the body of a patient.

The invention permits of drawings and the same are provided herein. It should be understood, however, that the use of the biocompatible polyimide or similar polymer provides the opportunity to use intramedullary plates and nails of virtually any shape and dimension, to provide for internal fixation, either by insertion into the marrow cavity of the bone (intramedullary nail) or by provision of a plate which may be used to anchor a fractured or damaged bone. It also permits to use self-tapping screws made of a biocompatible polyimide or similar polymer providing a completely metal-free system for patients. This not only offers important advantages for patients that are sensitive or allergic to metals, but eliminates the need for a second surgery to remove the stabilization materials.

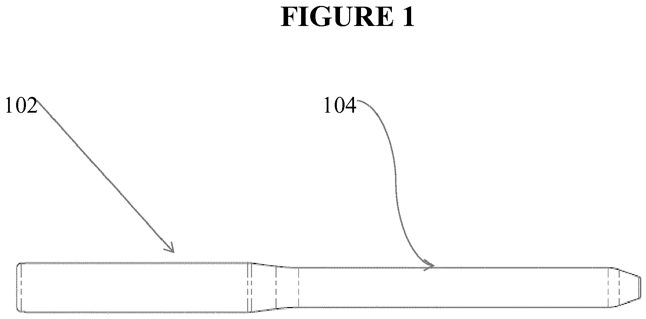

FIG 1 illustrates an intramedullary device of the invention, in this case, in the form of an elongated shaft or nail 104. This shaft is designed to be inserted into the hollow (intramedullary) marrow tunnel of a long bone, like a shin bone, but may be formulated in the shape of a plate, as may be necessary to provide bone fixation. The shaft 104 need not, but may be provided with a head or widened area 102 at one point with an inner thread, if it facilitates insertion into the shaft. Head 102 forms, in effect, a flange by which the nail may be grasped and manipulated. Importantly, shaft 104 is free of predrilled holes. Because conventional bone screws may be inserted into the shaft at any point along its length and at any angle, and from there screwed into the bone to fasten and stabilize the fractured bone, no predrilled holes are required.

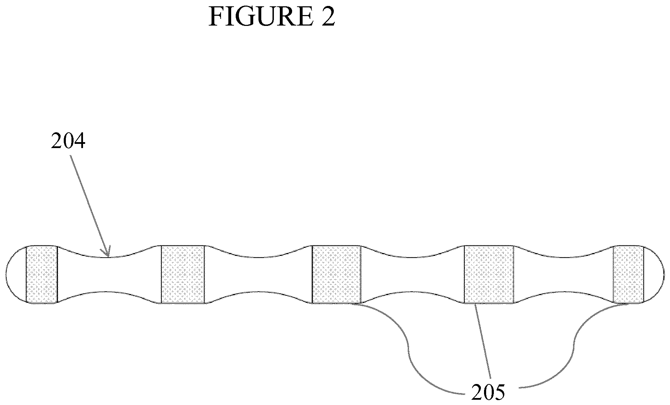

FIG 2 illustrates a similar intramedullary device (plate) of the invention. In this case, the plate has a wave-like marginal structure. The wide areas 205 are abraded and serve as "starters" to make the process of screwing the bone screw into the shaft easier. The thinner parts 204 have non-abraded smooth surface. The surgeon may feel the difference between the abraded rough area and the smooth area and thus be able to direct the drill to the right spot. If provided all along the length of the shaft at about the centerline, the surgeon is provided with a full range of solutions so as to optimize stabilization of the fracture.

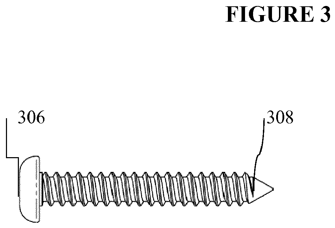

FIG 3 illustrates the bone screw made of polymer. The screw is provided with a self-tapping thread 308 and a drive head 306. Since polyimide is thermosetting cross-linked polymer, the screw and the nail will never cold fuse. This permits the provision of a completely metal-free device and method, avoiding the need to remove any bone fixation materials or devices following a successful resolution

A feature of the invention disclosed herein is an undisclosed advantage of the polyimide polymer MP-1™, or a similar biocompatible polymer that has the tensile strength and elasticity modulus and cross-linking exhibited by MP-1™. The replacement of conventional metallic intramedullary with a biocompatible polymer with physical characteristics not very different from those of bone itself leads to a number of improvements that are achieved simultaneously.

Considered from the point of view of the process of bone stabilization by use of the intramedullary nail of the invention, a first improvement or advantage of the invention is elimination of the need for metal scaffolding or braces to fix the nail in position. This allows the doctor or surgeon to proceed with a much smaller incision (minimally invasive). Since long bone fractures often occur in the arms and legs, which are frequently visible even when fully clothed, reducing the scarring and cosmetic imperfections arising from the operation reflects an important improvement.

Possibly the most dramatic improvement is achieved by eliminating the need for preset or predrilled holes in the nail or plate for the bone screws. Polyimide polymers like MP-1™ can receive screws anywhere along their length. Thus, the surgeon can affix the intramedullary nail 104 without regard to the preset screw holes - instead opting for locations along the bone that make the most sense for the specific patient being treated. The nail may be made more manageable by the provision of a head or handle 102, but the same is not required. The nail will be first drilled with a smaller hole suitable to the core dimension of the screw and then the self-tapping head 308 of the screw can be inserted through the cortex of the bone and the nail. This ability to personalize bone fixation to the needs of a particular patient and situation is unprecedented in current medicine. As an extension of the push to offer more and more personalized medicine, it represents a new advance in this practice. It also permits the use of the intramedullary nail fixation technique for situations where plates or casts were previously required - being lightweight and yet unlikely to be incompatible with the stresses experienced by the patient's bone and musculature, smaller bone fractures and displacements can be treated conveniently.

While, as detailed below, the device and method of the invention can be provided of biocompatible polymers like MP-1™, where necessary or preferred, this invention is consistent with the use of Kirschner wires (K-wires). Inserted K-wires can be provided with a polymer matrix, like the biocompatible polymer MP-1™, to which the wire will weld, providing a secure and enhanced fixation.

In a preferred embodiment, the intramedullary plate of the invention is provided with roughened the areas 205 for drilling by abrasion of the plate along its length. The areas 204 between holes will be left smooth. In this way the doctor can "feel" the difference in surface finish and direct the drill into the right location. While the plates, nails and similar devices of this invention may be of any size and shape suitable, providing one with the margin of the plate being wavy will contribute to the plate's strength. The plate will be first drilled with a smaller hole suitable to the core dimension of the screw and then the self-tapping screw can be inserted through the plate and bone. The abrasions 205 provided along the length of the intramedullary plate of the invention facilitate precise fixation of the plate to the bone, wherever, and importantly from whatever angle best serves the patient and the surgeon's needs. The operation is faster, more specifically tailored to the needs of the patient, and unlikely to cause the splintering or fracturing of bone material encountered in the prior art due to the use of metals of very high elastic modulus.

The same employment of a polyimide biocompatible polymer like MP-1™ solves a problem posed in the prior art - the need for imaging technologies to verify the correct placement of the nail and the bone screws. Not only does the need to image the placement of the device slow the operation and necessarily prolong the time spent under general anesthesia, increasing the risk to both the patient and the medical team, but repeated or continuous X-ray exposure is unhealthy for all individuals involved, both the patient and those medical personnel necessarily involved and exposed. By using a strong polymer like MP-1™ which has the strength to provide stabilization, holes can be drilled into the intramedullary nail or plate without the generation of excess heat along its length, and this drilling compatible with the stresses and forces applied to the bone itself. Thus, the process of bone repair, from initial incision to insertion to stabilization, the entire operation and related processes involved in bone fracture repair is improved, simplified and made significantly safer. There is no need to remove the structure once healing is complete, further simplifying the process. The debris created during drilling can easily be removed by sucking. Even if some debris is left in the body they are inert and do not cause any inflammation (particle sizes are more than 2 microns or so).

MP-1™ and similar polymers are biocompatible and shown to be safe, and need not be removed from the body at a point when the bone previously broken has healed. Prior art techniques that required brackets or similar metal supports or "scaffolding" to maintain the position of the bone are rendered unnecessary. No second operation to remove the metal structures is required. This reduces the overall cost of the operation, maximizes convenience and improves patient cooperation without any sacrifice in the character or result of the operation.

Replacement of metal devices implanted or inserted into the body with biocompatible polymers like MP-1™ provides additional health benefits. Replacing metal with a biocompatible polyimide will eliminate the problem of metallosis encountered in connection with prior art intramedullary nails and similar braces, plates and devices. Particularly in situation where bones bear on metal inserts, there is a tendency for metal debris to chip off or corrode, presenting a number of health risks as well as the potential for metal poisoning. Thus, the intramedullary nail of the invention is effective to facilitate bone repair with less inconvenience and cost, less difficulty to the surgeon, no risk of X-ray exposure and reduced risk of secondary health risks. The bone healing is completed in less time, without the need for a second operation for removal of the device, which means substantial gains in improvement of patient safety.

A particularly important aspect of the combination of reduced cost and risk coupled with increased patient comfort and safety means the traditional method used to stabilize large bones can be expanded to use in situations where prior art techniques could not be used in the stabilization of smaller bones, for partial distal breaks, and the like. The devices used in these procedures more closely resemble plates or flanges that secure the bone and protect it, rather than penetrating along its length. These advantages are secured without loss of the ability to provide a bone fixation/repair method that permits rapid weight bearing for the patient.

The invention is not limited to any specific shape, character or embodiment. The invention addressed in this application includes both the improved bone stabilization device, made of a biocompatible polyimide polymer with approximately the same modulus as bone, and the improved process for bone stabilization described above. It is broadly described in terms of specific shapes and features, but alternate shapes and features will occur to those of ordinary skill in the art without the exercise of inventive faculty.

## Claims

1. A bone stabilization device for insertion in the body of a patient requiring bone stabilization, said bone stabilization device comprising:
a shaft comprised of a formable, pyromellitic, dianhydride (PMDA)-free, non-halogenated, aromatic polyimide thermosetting polymer which preferably exhibits approximately the tensile strength and stiffness of human bone, wherein said shaft is of a shape suitable for stabilization of said bone requiring stabilization, and is free of predrilled holes for bone screws.

2. The bone stabilization device of claim 1, wherein said shaft is of a shape and size to be inserted in the intramedullary marrow tunnel of a human bone, and is susceptible of being secured to the bone it stabilizes by bone self-tapping screws.

3. The bone stabilization device of claim 1, wherein said device is of a shape and size suitable to stabilize a partial fracture and is in the form of a plate or flange attached to the bone but not penetrating any cavity of the bone.

4. The bone stabilization device of any one of the preceding claims, wherein said device comprises abrasion roughening at points along its length to aid in the driving of screws to secure said device to said bone to be stabilized.

5. A kit comprising:
the bone stabilization device of any one of the preceding claims;
bone self-tapping screws that may be screwed at any point along the length and at any angle with respect to said device.

6. The kit of claim 5, wherein said kit is free of scaffolding or support elements necessary to hold said device in place relative to said bone.

7. The kit of claim 5 or claim 6, wherein said device is in the form of an intramedullary nail for insertion into the intramedullary marrow tunnel of the bone to be stabilized, and wherein said nail is securable by said bone self-tapping screws screwed through said nail and into said bone cortex to be stabilized.

8. The kit of claim 5 or claim 6, wherein said device is in the form of a plate or flange affixable to bone along said bone, so as to stabilize said bone while it heals.

9. The kit of any one of claims 5 to 8, wherein said bone self-tapping screws are prepared from a biocompatible polymer which need not be removed from the body.

10. The kit of any one of claims 5 to 9, wherein said kit does not comprise any materials of metal to be left in the body of a patient.
